# EUROPEAN PATENT SPECIFICATION

(11) **EP 1 848 480 B1**
(45) Date of publication and mention of the grant of the patent: **27.10.2010**
(21) Application number: 06720659.9
(22) Date of filing: 10.02.2006
(51) Int. Cl.: A61M 5/30

(54) **SURFACE INJECTION DEVICE**
OBERFLÄCHENINJIZIERUNGSVORRICHTUNG
DISPOSITIF D'INJECTION A TRAVERS UNE SURFACE

(30) Priority: 11.02.2005 US 652483 P
(43) Date of publication of application: 31.10.2007
(73) Proprietor: Massachusetts Institute of Technology, Cambridge, MA 02139 (US)
(72) Inventor: HUNTER, Ian, W., Lincoln, MA 01773 (US); TABERNER, Andrew, J., Lexington, MA 02420 (US); HEMOND, Brian, D., Lexington, MA 02420 (US); WENDELL, Dawn, M., Farmington, CT 06032 (US); BALL, Nathan, B., Boston, MA 02215 (US); HOGAN, Nora, Catherine, Boston, MA 02111 (US)
(74) Representative: Jenkins, Peter David
(86) International application number: PCT/US2006/004898
(87) International publication number: WO 2006/086719

(56) References cited:
- DE-A- 10 146 535
- DE-U1- 20 105 183
- US-A- 3 788 315
- US-A- 5 074 843
- US-A1- 2002 055 729
- US-A1- 2002 095 124

## Description

### BACKGROUND

Injection of a liquid such as a drug into a human patient or an agriculture animal is performed in a number of ways. One of the easiest methods for drug delivery is through the skin, which is the outermost protective layer of the body. It is composed of the epidermis, including the stratum corneum, the stratum granulosum, the stratum spinosum, and the stratum basale, and the dermis, containing, among other things, the capillary layer. The stratum corneum is a tough, scaly layer made of dead cell tissue. It extends around 10-20 microns from the skin surface and has no blood supply. Because of the density of this layer of cells, moving compounds across the skin, either into or out of the body, can be very difficult.

The current technology for delivering local pharmaceuticals through the skin includes methods that use needles or other skin piercing devices. Invasive procedures, such as use of needles or lances, effectively overcome the barrier function of the stratum corneum. However, these methods suffer from several major disadvantages: local skin damage, bleeding, risk of infection at the injection site, and creation of contaminated needles or lances that must be disposed of Further, when these devices are used to inject drugs in agriculture animals, the needles break off from time to time and remain embedded in the animal. Thus, it would be advantageous to be able to inject small, precise volumes of pharmaceuticals quickly through the skin without the potential of a needle breaking off in the animal.

DE 20105183 discloses an ampoule for a needle free injection mechanism having a chamber for the uptake of the medium to be injected. The ampoule also has a bottom with an opening having several individual openings with channels guided through the bottom chamber. The multiple openings allow a larger quantity of medium to be injected in a one-time injection.

US 2002/055729 discloses a catheter including a shaft having an infusion lumen extending therethrough, wherein the proximal end of the shaft is connected to a pressurised fluid source. The distal end of the shaft includes a nozzle having an injection port in fluid communication with the infusion lumen such that fluid from the pressurised fluid source may be delivered to the heart tissue at a sufficiently high exit velocity to partially penetrate the heart tissue.

### SUMMARY

According to aspects of the present invention there is provided a needle free transdermal transport device according to independent claim 1. According to another aspect of the present invention there is provided the method of manufacturing a nozzle adapted to transfer a substance non-axially to a surface of a body according to independent claim 8. Preferred features are defined in the dependent claims.

The present invention relates to devices for transferring a substance across a surface of a biological body. The needle-free transdermal transport device includes a reservoir for storing a substance. Also the device includes a nozzle, which is in fluid communication with the reservoir. The nozzle is adapted to be pressed against and depress without piercing, the surface of the biological body. The nozzle includes a lateral aperture through which the substance is laterally injected. The device also includes an actuator, which is in communication with the reservoir. The actuator is adapted to generate a pressure within the reservoir when activated, thereby inducing injection of the substance laterally through the aperture into the biological body by piercing the surface of the biological body.

The actuator may be any suitable type of actuator. For example, the actuator can be a linear actuator. The actuator can also be an electromagnetic actuator, such as a Lorentz force actuator, which comprises a magnetic force used to generate pressure within the reservoir. Other types of actuators such as spring loaded actuators, shape memory actuators, electric motor actuators, and gas generation actuators can also be used with the device. The actuator can be controllable during an actuation in order to vary the pressure applied within the reservoir.

The nozzle includes a tube with a closed end having apertures through the side of the tube. The tube can be closed with a plug having an inner surface that directs flow to the apertures. The nozzle can include a plurality of lateral apertures. This allows the transferring of the substance to a preferred region of the body. The nozzle is adapted to produce a shallow injection with respect to a surface; when the axis of the nozzle is perpendicularly aligned with a surface of the body. A retractable shroud can be in communication with the nozzle.

The substance can be injected through a plurality of lateral apertures in the nozzle. Also, the substance can be injected into a single layer under the surface of the body. The substance can also be injected into a plane defined between different layers of the surface of the body.

The disclosure is also directed to a method of manufacturing a nozzle, which is adapted to non-axially transfer a substance to a surface of a body. The steps include first providing an elongated member defining a central lumen. Next, one end of the elongated member is positioned such that it is in close proximity with an Electro Spark Discharge (ESD) wire. Finally, the ESD wire is energized to vaporize the end of the elongated member, thereby forming a non-axial aperture that is adapted to transfer the substance to the surface of the biological body.

In order to form an elliptical aperture on the elongated member, a linear ESD wire is used during vaporization. A preferred orientation of a major axis of the elliptical aperture can be obtained by controlling the alignment of the ESD wire and the elongated member. If a plurality of apertures is desired, different portions of the end of the elongated member can be repeatedly vaporized with an ESD to form an array of apertures.

### BRIEF DESCRIPTION OF THE DRAWINGS

The foregoing and other objects, features and advantages of the invention will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention.
FIG. 1 shows an embodiment of a surface injection device;
FIGS. 2A-2C illustrate an exemplary nozzle including lateral apertures;
FIGS. 3A-3B illustrate an exemplary nozzle including uniformly and radially distributed apertures;
FIG. 4 illustrates an exemplary nozzle with lateral apertures angled to produce a shallow injection;
FIG. 5 illustrates a method of producing a shallow injection using a surface injection device;
FIG. 6 illustrates a method of producing an injection between layers of the skin using a surface injection device;
FIG. 7 illustrates an exemplary surface-injection nozzle with a retractable shroud;
FIG. 8 illustrates a surface injection device used with an electromagnetic actuator;
FIG. 9 illustrates a surface injection device used with a shape memory alloy actuator;
FIGS. 10A-10B illustrate a surface injection device used with an alternative shape memory alloy actuator;
FIG. 11 illustrates a method of manufacturing a nozzle of a surface injection device;
FIG. 12 shows further detail of the method of manufacturing of FIGS. 11.
FIGS. 13A-13C illustrate exemplary nozzles including 6, 8, and 12 aperture arrays;
FIG. 14 shows a plug used in conjunction with a nozzle;
FIG. 15 illustrates an exemplary handheld, portable injection device; and
FIG. 16 shows an array of nozzles.

### DETAILED DESCRIPTION OF THE INVENTION

A description of preferred embodiments of the invention follows.

A transdermal transport device, or injection device, is configured to transfer a substance across a surface of a biological body. Injection devices include devices having one or more needles configured to pierce the skin prior to injection of the substance (e.g., typical hypodermic needle). Other injection devices are configured to inject a substance beneath the skin without first piercing the skin with a needle (i.e., needle-free). It should be noted that the term needle-free as used herein refers to devices that inject without first piercing the skin using a needle. Thus, needle-free devices may include a needle, but the needle is not used to first pierce the skin. Some needle-free injection devices rely on a pioneer projectile ejected from the device to first pierce the skin. Other needle-free injection devices rely on pressure provided by the drug itself.

Injection devices generally include a reservoir or chamber for storing a substance to be injected (e.g., a drug). Injection devices also include a distal port through which the drug can be expelled to enter the body. The reservoir is typically in fluid communication with the distal port through a lumen. In operation, a pressure is applied to the reservoir forcing the drug through the lumen and out of the distal port. For needle-free applications, the distal port generally forms a nozzle through which the drug is expelled, forming a jet. The velocity of the jet can be sufficient to pierce the outer-most layer of skin and to penetrate the body to a desired depth. Further details on needle-free injection devices can be found in the U.S. Application titled "Controlled Needle-Free Transport", filed concurrently on February 10, 2006, claiming the benefit of U.S. Provisional Application No. 60/652,483 filed on February 11, 2005.

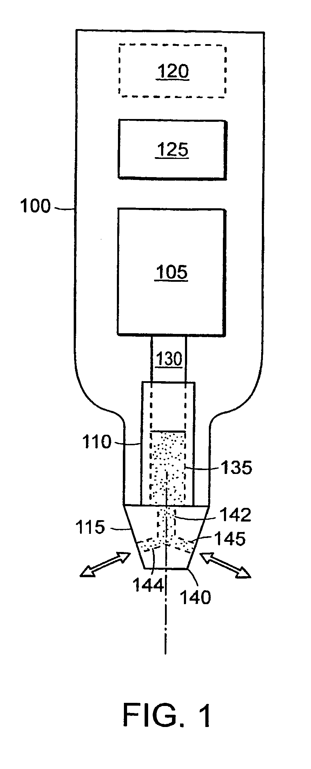

FIG. 1 shows a surface injection device 100. The surface injection device 100 includes a nozzle 115. The nozzle 115 is adapted for perpendicular alignment with the surface of a biological body. The nozzle 115 includes a distal end 140 defining one or more lateral apertures 145 for dispensing a substance. The shape of the nozzle 115 can be cylindrical, spherical, frustoconical, or any suitable shape that adapts to the surface of a biological body.

A reservoir 110 is in fluid communication with the nozzle 115 through an axial lumen 142 along a longitudinal axis of the nozzle 115, the axial lumen 142 being part of the nozzle 115. The reservoir 110 holds the substance 135 to be injected. Alternatively, any suitable means of holding the substance 135 can be used, such as a chamber, a syringe, or an expandable bellows chamber. The surface injection device 100 also includes a piston or plunger 130, which is in communication with the reservoir 110.

The surface injection device 100 further includes an actuator 105. The actuator 105, when activated is adapted to advance the piston 130 distally, such that a sufficient amount of pressure is applied to the substance 135 within the reservoir 110. When the actuator 105 is inactivated, the piston 130.can be drawn to its original inactive position, or remain fixed at its current position. The actuator 105 may be, for example, a linear actuator, an electromagnetic actuator such as a Lorentz Force actuator, a shape memory alloy actuator, a spring loaded actuator, a gas generation actuator, or any suitable actuator to actuate the piston 130 to apply sufficient pressure to expel the substance 135 from the reservoir 110.

The substance 135 can flow from the reservoir 110 through the axial lumen 142 into the nozzle 115. The nozzle 115 also includes non-axial lumens 144. The non-axial lumens 144 are in fluid communication with and connect the axial lumen 142 to the lateral apertures 145. Alternatively, an axial lumen 142 may not be included, and the distal end of the reservoir 110 may be directly connected to the non-axial lumens 142.

The surface injection device 100 may include a power source 125. The power source 125 may be, for example, a battery, a storage capacitor, a connection to an electrical supply line, or any power source capable of providing sufficient activation power to operate the surface injection device 100. In some embodiments, the surface injection device 100 also includes a controller 120. The controller 120 may be user controlled. In one embodiment, the controller 120 is a simple switch that may be manually operated by a user, for example a push button. Alternatively, the controller 120 may be automatically operated. The controller 120 may control current flow from the power source 125 to the actuator 105.

In other embodiments, the controller 120 may allow actuation only when certain parameters are met. For example, the controller 120 could initiate an injection only if enough energy remains in the power source 125 to conclude the injection.

Alternatively or in addition, the controller 120 may be configured to determine adequate dosage of the substance 135 to be delivered, based on certain parameters. The parameters, for example, can include stored values, such as an expiration date code of the drug, or information obtained from a remote source. The surface injection device 100 may, for example, be equipped with a communications interface, which interrogates a subject prior to injection. Alternatively or in addition, the surface injector 100 may query a remote database to determine parameters regarding application or dosage, and the controller 120 may initiate activation based on the parameters.

In still other embodiments, the controller 120 can include a servo-controller that incorporates feedback. For example, the controller 120 may receive output of a force transducer suitably placed to sense the force being applied to the substance stored in the reservoir 110. When the skin is penetrated, there may be a sudden fluctuation in the sensed force as the pressure within the reservoir 110 varies. That fluctuation in pressure may be provided to the controller 120 and used to alter the applied current (i.e., the force) thereby transitioning from a piercing phase requiring a higher current, to a delivery phase requiring a lower current. The controller 120 may perform a variety of suitable operations, all of which are within the scope of this invention.

In operation, a substance 135 is first loaded into the reservoir 110. The substance 135 can be loaded into the reservoir 110 by coupling an inlet port to the reservoir 110 through a valve, which when opened, allows the substance 135 to flow into the reservoir 110. Alternatively, the substance 135 can simply be drawn into the nozzle 115. The reservoir 110 may also be a replaceable reservoir 110 with a preloaded substance. The substance 135 can be loaded into the reservoir 110 by any suitable means.

The surface injection device 100 is positioned on the surface of the biological body. The controller 120 is then activated either by a user or automatically to initiate the injection process. Once the controller 120 initiates the process, the actuator 105 is activated. The actuator 105 causes the piston 130 to advance distally. The piston 130, therefore, applies pressure to the substance 135 within the reservoir 110, causing the substance 135 to flow at a sufficient velocity through the axial lumen 142 connecting the reservoir 110 and the nozzle 115.

Once the substance flows through the axial lumen 142, it is directed to flow through the non-axial lumen 144. The non-axial lumen 144 directs the substance 135 through a lateral aperture or apertures 145 disposed on the nozzle 115, and transfers it non-axially to the surface of the biological body. The surface of the biological body is pierced by the velocity of the expelled substance 135, and the substance 135 is non-invasively delivered through the piercing. The controller 120 is then inactivated, because the delivery of the substance 135 is complete. Alternatively, the surface injection device 100 can be used to draw a substance from a biological body, as opposed to injecting a substance into a biological body.

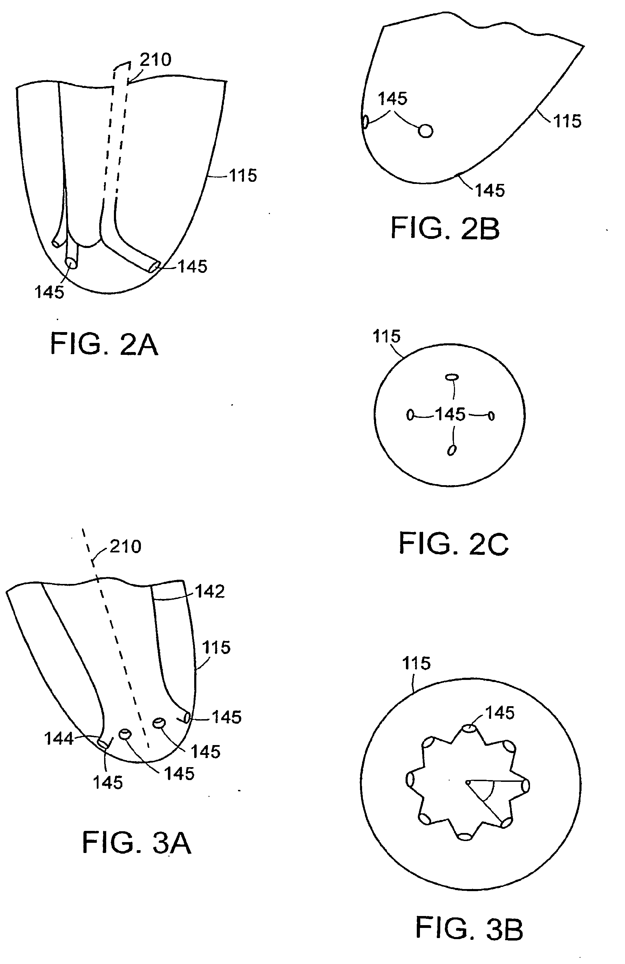

Details of the lateral apertures 145 on the nozzle 115 are shown in FIGS. 2A-2C. An array of apertures 145 can be distributed laterally about the distal end of a nozzle 115. The apertures 145 can be circular, elliptical, or any suitable shape. For example, six apertures 145 can be uniformly distributed about the distal end of a nozzle 115. As shown in FIGS. 2A and 2B, the apertures 145 are distributed uniformly and radially about a longitudinal axis 210 of the nozzle 115. The uniform distribution of the apertures 145 allows for an even expelling of the substance 135 to the biological body. Alternatively, the apertures 145 need not be uniformly distributed, and may be arranged in any appropriate pattern to produce an injection. A bottom view of the nozzle 115 with uniformly distributed apertures 145 is shown in FIG. 2C.

An array of eight uniformly distributed apertures 145 is shown in FIGS. 3A and 3B. The axial lumen 142 parallel to the longitudinal axis 210 is clearly show in FIG. 3A. The axial lumen 142 spreads at its distal end to form a series of non-axial lumens 144, connecting axial lumen 142 to apertures 145. The apertures 145 are, therefore, formed on a non-axial facing surface of the nozzle 115.

FIG. 3B shows a bottom view of the nozzle 115 with uniformly distributed apertures 145 about the longitudinal axis 210. Each aperture 145 has an equivalent angular and radial separation. This is beneficial in ensuring an even expelling of the substance 135 to the surface of the biological body.

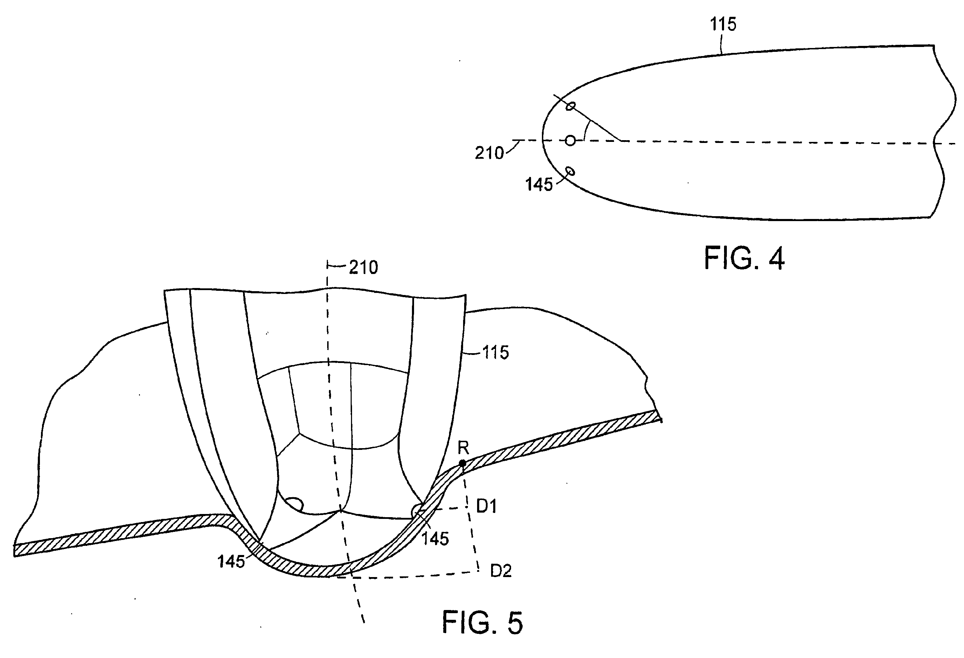

In one embodiment, the surface injection device 100 can be used to make very shallow injections within the surface of the biological body. As shown in FIG. 4, this can be accomplished by positioning the apertures 145 at a larger angle with respect to the longitudinal axis 210 of the nozzle 115. For the purpose of a shallow injection, the apertures 145 are preferably positioned at an angle of at least 45 degrees with respect to the longitudinal axis 210. Positioning the apertures 145 at larger angles with respect to the longitudinal axis 210 allows the substance 135 to be expelled substantially parallel to the surface of the biological body, thereby producing a shallow injection.

As shown in FIG. 5, to produce a shallow injection, the nozzle 115 is brought into substantially perpendicular alignment with the surface of the biological body. The distal end of the nozzle 115 is depressed below a reference point (labeled as R) within the skin of the biological body, to a depth D2 with respect to the reference point R. Preferably, the distal end of the nozzle 115 is bluntly shaped such that it can easily depress the skin without piercing the skin. The apertures 145, distributed on the nozzle 115 about the longitudinal axis 210 and positioned preferably at an angle of more than 45 degrees with respect to the longitudinal axis 210, are depressed to a shallower depth, D1. When injecting, the nozzle 115 is positioned non-orthogonally to the relaxed surface of the skin, the relaxed surface being the non-depressed portion of the skin.

As described in FIG. 1, the surface injection device 100 can then be activated and an actuator 105 can be used to force the substance 135 from the reservoir 110 through the axial lumen 142, the material exiting the non-axial lumen 144 through the lateral array of apertures 145 and penetrating the skin. Thus, a relatively shallow injection within the skin of the biological body is produced.

Beneficially, the angle of the apertures 145 can be varied with respect to the longitudinal axis of the nozzle 115 during manufacture. For example, if a deeper injection is desired, the apertures 145 can be positioned at a smaller or acute angle with respect to the longitudinal axis 210 of the nozzle 115. This would result in the injection being made substantially perpendicular to the skin's surface and would thus result in a deeper injection of the substance 135.

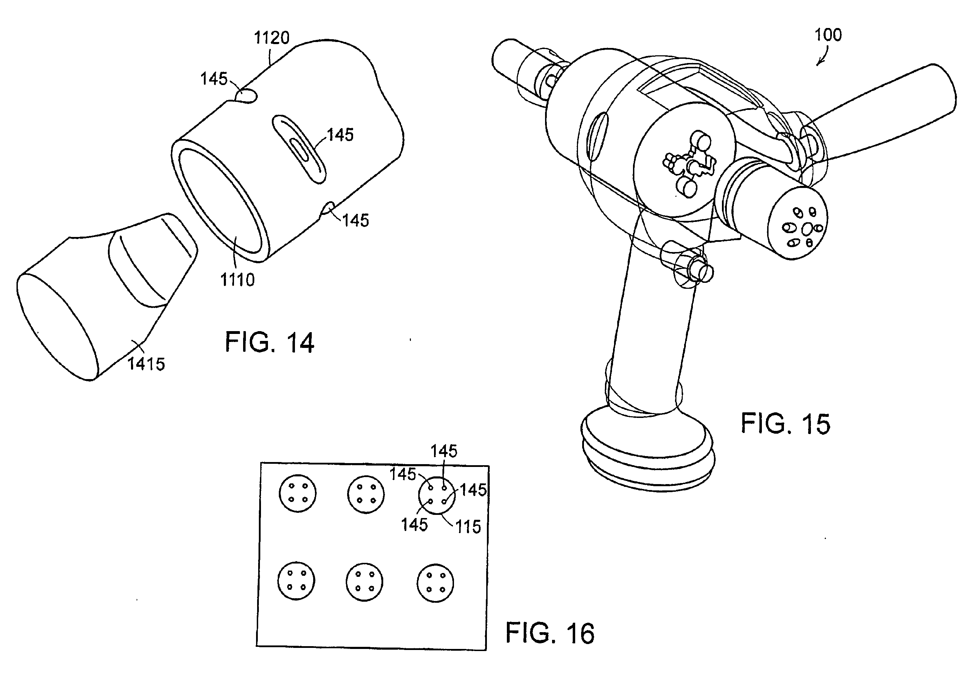

Alternatively, or in addition, the surface injection device 100 can include more than one nozzle 115 as shown in FIG. 16. For example, the nozzles 115 can be configured in an array adapted to cover an even greater surface area of the skin. The array can be a two-dimensional array, such as an N x N rectangular arrangement of nozzles 115. Thus, injection patterns formed by each of the respective nozzles 115 are combined to produce an overall injection pattern, for example, a 4 x 4 array of rosette patterns. The multiple nozzles 115 of the array can be actuated in parallel, all injecting at substantially the same time, or alternatively in a series with one or more nozzles 115 injecting in sequence one after another.

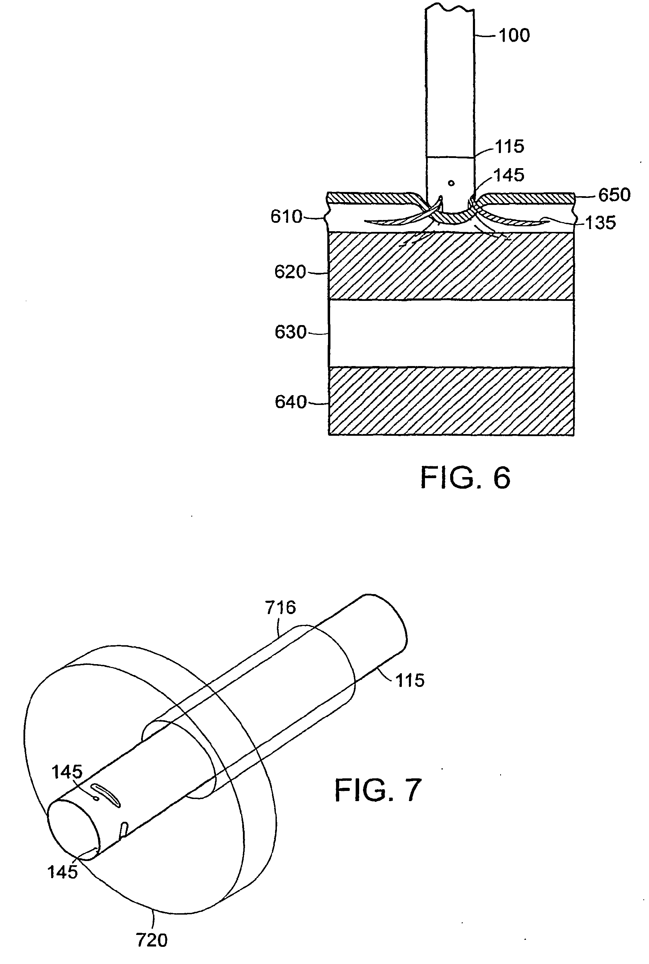

Needle-free injectors have commonly been directed to inject perpendicularly into the skin surface. By injecting the substance 135 through laterally directed apertures 145, a wider distribution of the substance 135 can occur. Additionally, a significant volume of the substance 135 can be kept closer to the surface, thus producing shallower injections, as shown in FIG. 6.

The skin has three main layers. The top most layer is the epidermis 610, which is translucent. The epidermis includes the stratum corneum, the stratum granulosum, the stratum spinosum, and the stratum basale, and the dermis, containing, among other things, the capillary layer. The stratum corneum 650 is a tough, scaly layer made of dead cell tissue. It extends around 10-20 microns from the skin surface and has no blood supply. Because of the density of this layer of cells, moving compounds across the skin, either into or out of the body, can be very difficult. The second later is dermis 620, which contains blood vessels, nerves, hair roots and sweat glands. Below the dermis lies a layer of fat, the subcutaneous fat 630. The subcutaneous fat lies on the muscles 640 and bones, to which the whole skin structure is attached by connective tissues.

The surface injector 100 can deliver the substance to whichever layer of the skin is desired, by simply varying the angle of the apertures 145 with respect to the longitudinal axis of the nozzle 115. By knowing the thickness of each layer, the surface injector 100 can be manipulated to inject the appropriate layer. This can be done as stated, by either varying the angle of apertures 145, by varying the depth that the nozzle 115 is pressed within the skin surface, or by a combination of these factors.

Alternatively, or in addition, the surface injector 100 can be used to apply the substance 135 selectively between layers of the skin and beneath the skin. For example, as shown here, the injection is being made in a plane between the epidermis 610 and the dermis 620. Beneficially, this allows the substance 135 to spread into both layers.

By delivering the substance 135 into a plane at a desired depth beneath the skin surface of a biological body, the surface injector 100 has commercial applications for treating conditions of the skin and selective layers of tissue beneath the skin. For example, the surface injection device 100 can be used to inject a collagenese enzyme mix into sheep to eliminate blow-fly strike disease -- a particularly vexing problem to the merino sheep in Australia. The collagenese enzyme breaks down tissues beneath the skin to reduce or eliminate folds within the skin that are particularly susceptible to the blow fly parasite. Other applications include a similar injection of a collagenese enzyme mix into burn patients to aid in reducing the effects of scarring.

As shown in FIG. 7, the nozzle 115 can be provided in combination with a retractable protective sleeve or shroud 710. The retractable shroud 710 in a rest position preferably covers the apertures 145 to prevent contamination and leakage, and to facilitate refilling of the surface injection device 100. When retracted, the one or more apertures 145 of the nozzle 115 are exposed to operate as described previously. The shroud 710 can be retracted axially, being drawn in a proximal direction (i.e., away from the skin surface) with respect to the distal end of the nozzle 115. The shroud 710 may be slidable upon the nozzle 115 allowing it to advance distally and proximally with ease. The body of the shroud 710 can include any suitable locking mechanism to lock the shroud 710 on the nozzle 115 in the desired position. Alternatively, or in addition, the shroud 710 can be rotated to selectively expose the one or more apertures 145.

For example, the shroud 710 can include complementary apertures corresponding to the one or more apertures 145 of the nozzle 115. In a first position, the one or more apertures 145 of the nozzle 115 are covered by the shroud 710. When rotated, the complementary apertures of the shroud 710 are brought into at least partial alignment with the one or more apertures 145 of the nozzle 115 to expose at least a portion of the apertures 145. Shapes and/or alignments of the complimentary apertures can be used in combination with shapes and/or alignments of the one or more apertures 145 of the nozzle 115 to control dimensions of one or more resulting apertures defined by the overlapping of both apertures.

The shroud 710 can include a distal flange or protective sleeve 720 adapted to engage the skin surface surrounding an injection site. When the protective sleeve 720 is advanced to a position at which it abuts the skin surface, the shroud 710 is inhibited from further advancement by the skin surface. With the nozzle 115 slideably engaged with the shroud 710, the nozzle 115 may be advanced further beyond the end of the protective sleeve 720. Thus, apertures provided within the nozzle 115 that are initially covered by the shroud 710 and/or the protective sleeve 720, can be exposed by sliding the nozzle 115 beyond the end of the protective sleeve 720.

The shroud 710, may for example, be spring loaded, thus allowing the retraction of the shroud 710 when the protective sleeve 720 comes in contact with the skin. Alternatively, any suitable means for allowing facilitated retraction may be used with the shroud 710.

Additionally, the protective sleeve 720 can form a skin-surface reference plane useful for controlling a depth of advancement of the nozzle 115. For example, the surface injection device 100 can include a stopping means that inhibits further advancement of the nozzle 115 beyond a predetermined distance measured relative to the end of the protective sleeve 720.

Alternatively or in addition, the surface injection device 100 can include a position sensor for sensing the position of the nozzle 115 relative to the end of the protective sleeve 720. For example, the positioning sensor can be placed such that it measures the displacement of the proximal end of the shroud 710 with respect to the nozzle 115. Thus, prior to nozzle 115 being advanced into the skin of the biological body, the proximal end of the shroud 710 would be at a position P1. When the nozzle 115 is pressed into the skin, the shroud 710 would retract as a result of the contact of the skin with the protective sleeve 720. The proximal end of the shroud 710 would thus be retracted to a position P2. The position sensor could measure the distance between P1 and P2. The distance would indicate the depth or advancement of the nozzle 115 within the skin.

Alternatively, a position sensor may not be necessary. The nozzle 115 could simply be marked with measurement graduations to measure the displacement of the shroud. For example, graduations can be marked on the proximal end of the nozzle 115 to measure positions P1 and P2 as previously described. Any suitable position/distance measuring mechanism can optionally be incorporated with the shroud 710, the protective sleeve 720, the nozzle 115, or any part of the surface injection device 100.

As described with respect to FIG. 1, various types of suitable actuators may be used with the surface injection device 100. For example, the actuator 105 may be a spring loaded actuator. The spring loaded actuator is initially in a compressed state. A release means would allow the spring to expand thus advancing the piston 130 forward to apply pressure to the substance 135 to expel it out of the apertures 145 of the nozzle 115.

Alternatively, the actuator 105 may be an electric motor actuator. Therefore, the electric motor actuator could be activated to advance the piston 130 distally. Activation power for the electric motor can be provided by the optional power source 125.

Gas generator actuators may also be used, in which a high pressured gas is used to activate the actuator 105. For example, the actuator 105 may include an expandable reservoir 110 in communication with the piston 130, which expands with a high pressure gas, thus advancing the piston 130. Alternatively, a squib type gas actuator may be used. A squib type actuator, can cause an explosion which is used to generate a pressurized gas in order to advance the piston 130.

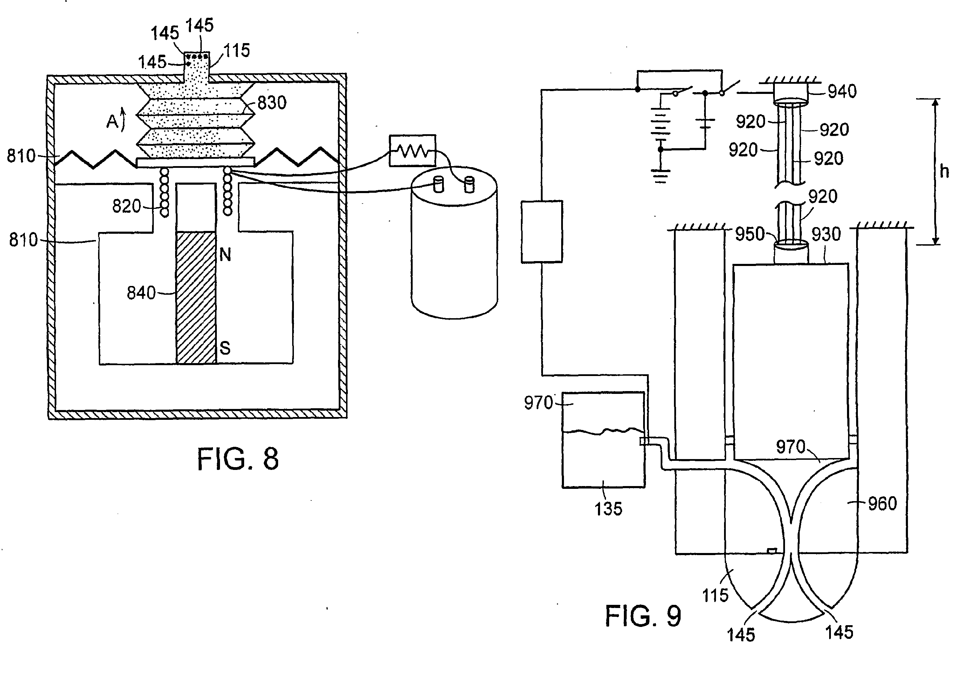

Alternatively, an electromagnetic actuator 810 can be used as shown in FIG. 8. The actuator 810 includes a conducting coil 820 disposed relative to a magnetic field, such that an electrical current induced within the coil 820 results in the generation of a corresponding mechanical force. The relationship between the magnetic field the electrical current and the resulting force being well defined and generally referred to as the Lorentz force law.

As shown here, the electromagnetic impulse pressure actuator 820 is coupled to an expandable bellows chamber 830. A current into the coil 820 provided by a power source 825, causes movement of the coil 820 in the direction of Arrow A. The current induced within the coil 820 in the presence of the magnetic field due to presence of a magnet 840, results in the generation of a proportional force directed perpendicular to both the magnetic field and the coil 820, as indicated by the Arrow A. Thus, the actuator 820 either compresses or expands the bellows chamber 830, depending upon the direction of the current. The nozzle 115 is in fluid communication with the bellows chamber 830 such that a formulation stored within the bellows chamber 830 is forced through the apertures 145 of the nozzle when the bellows 830 is compressed. Operation of the electromagnetic actuator 810 is both controllable and highly predictable given the physical properties of the electromagnetic actuator 810 and the input electrical current.

As described with respect to FIG. 1, the electromagnetic actuator 810 can be combined with a servo-controller to create a servo-controlled injection. An injection pressure is generated to respond in real-time to one or more physical properties (e.g., pressure, position, volume, etc.) determined by one or more sensors. The electromagnetic actuator 810 generates a high-pressure pulse having a rapid rise time (e.g., less than 1 millisecond) for injecting a formulation beneath the skin. The pressure provided by the controllable electromagnetic actuator 810 can be varied during the actuation of a single injection to achieve a desired result.

For example, a first high-pressure is initially provided to the formulation to penetrate the outer surface layer of a biological body. Once the skin is penetrated, the pressure is reduced to a second, lower pressure for the remainder of the injection. The servo-controller can be used to determine when the skin is penetrated by sensing a change in pressure within the chamber and to adjust the injection pressure accordingly, by controlling the amplitude of electrical current driving the controllable electromagnetic actuator 810.

An alternative type of actuator is shown in FIG. 9. The actuator 910 includes one to ten or more fibers 920 arranged parallel to one another. One end of the fibers 920 is attached to the surface 940 and one end to a piston 930 with another clamp 950 so that the fibers 920 are under tension. The fibers are composed of a shape memory alloy, such as Ni-Ti, available under the Trade Mark Nitinol. When a potential is applied to the ends, the shape memory fibers 920 contract to draw the piston 930 proximally. This allows the substance 135 to be released from a drug vial 970, flow into the nozzle 115, and out of the apertures 145, thus dispensing the substance 135. Shape memory alloy actuators are further described in U.S. Patent Application Serial No. 10/200,574, filed on July 19, 2002, and claiming the benefit of U.S. Provisional Application No. 60/338,169, filed on October 26, 2001.

In the presently discussed embodiment, the piston 930 and a tapered section 960 are permanent magnets such that the facing surfaces of the tapered section 960 and the end section 970 are oppositely polarized. When the potential is removed from shape memory fibers 920, the fibers relax thus allowing the piston 930 to be drawn to the tapered section 960, thus completing an injection cycle.

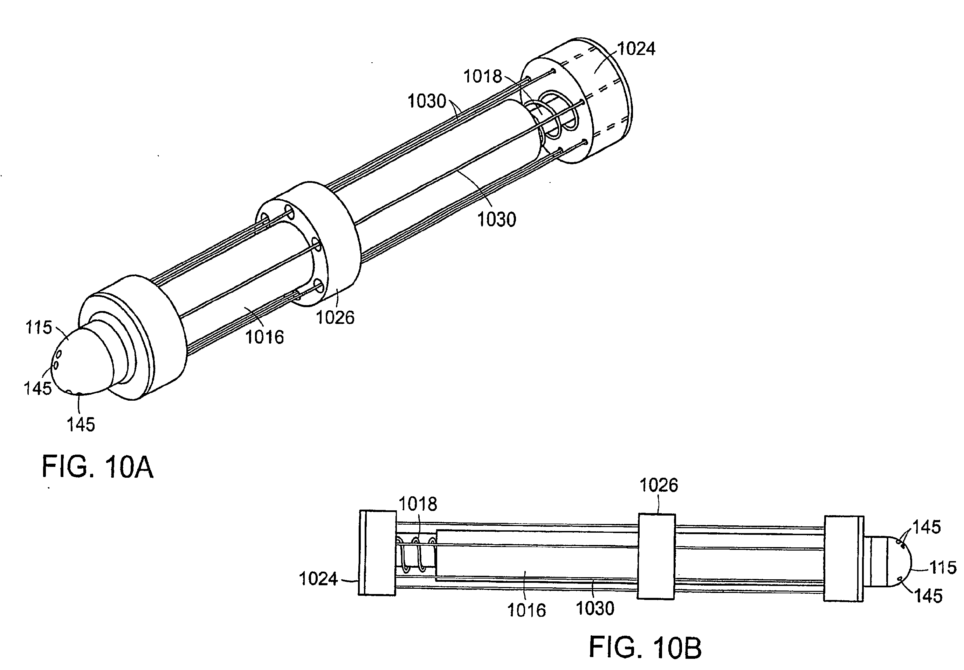

An alternative embodiment of the surface injection device 100 used with a shape memory type actuator is shown in FIGS. 10A-10B. The surface injection device 100 includes two electrical contact plates 1024 and 1026.

In addition to the contact plates 1024 and 1026, the actuator includes one to six or more wires 1030 positioned about the tube 1016 and parallel to one another. One of each wire 1030 is attached to the contact plate 1024. The contact plates 1024 and 1026 are electrically conductive.

The wires 1030 are made of a suitable material that contracts when heated and can be used as an actuation method, such as a shape memory alloy, as described in FIG. 9. Heating can be accomplished by passing a current through the wire 1030 through the contact plates 1024 and 1026. The larger contraction of shape memory alloys makes them desirable for the illustrated embodiment. The contraction of the wires 1030 causes a piston 1018 to be pushed towards the nozzle 115, hereby forcing the drug from the chamber out the apertures 145. Further details on shape memory type and other types of actuators using contracting materials are described in U.S. Patent Application Serial No. 10/657,734, filed on September 8, 2003, and claiming the benefit of U.S. Provisional Application No. 60/424,114, filed on November 5, 2002.

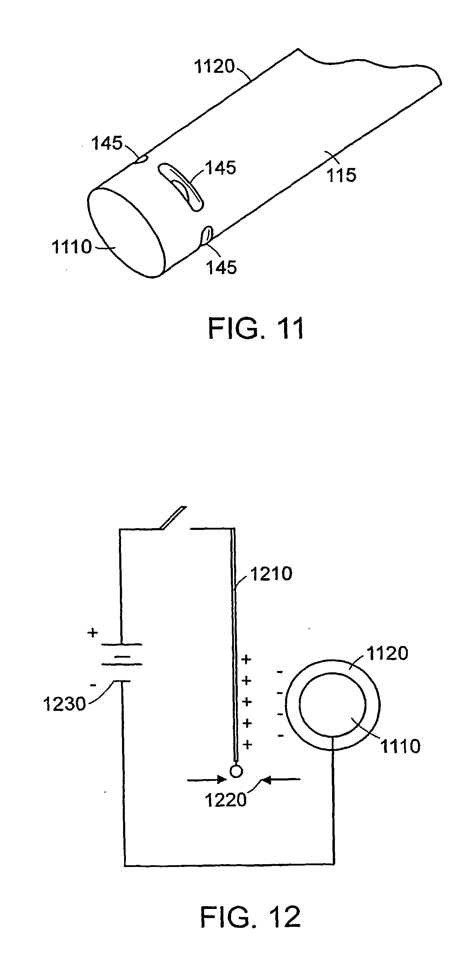

A method of manufacturing the nozzle 115 of the surface injection device 100 is shown in FIG. 11 and FIG. 12. A method for manufacturing the nozzle 115 includes providing an elongated member 1120 defining a central lumen 1110, such as an elongated hollow cylinder (e.g., a thin-walled stainless steel tube). Apertures 145 can be formed using Electro Spark Discharge (ESD) machining. As shown in FIG. 12, an end of the elongated member 1120 is brought into close proximity with an ESD wire 1210, leaving a gap 1220 between the ESD wire 1210 and the elongated member 1120.

The ESD 1210 wire is then energized by an electrical source 1230, creating an arc across the gap 1220. Thus, the gap 1220 acts like a spark gap, causing a proximate portion of an end of the elongated member 1120 to vaporize. Preferably, enough of the elongated member 1120 is vaporized to form an aperture 145 between the exterior and the central lumen 1110. A linear ESD wire can thus form an elliptical aperture 145 in the elongated member 1120.

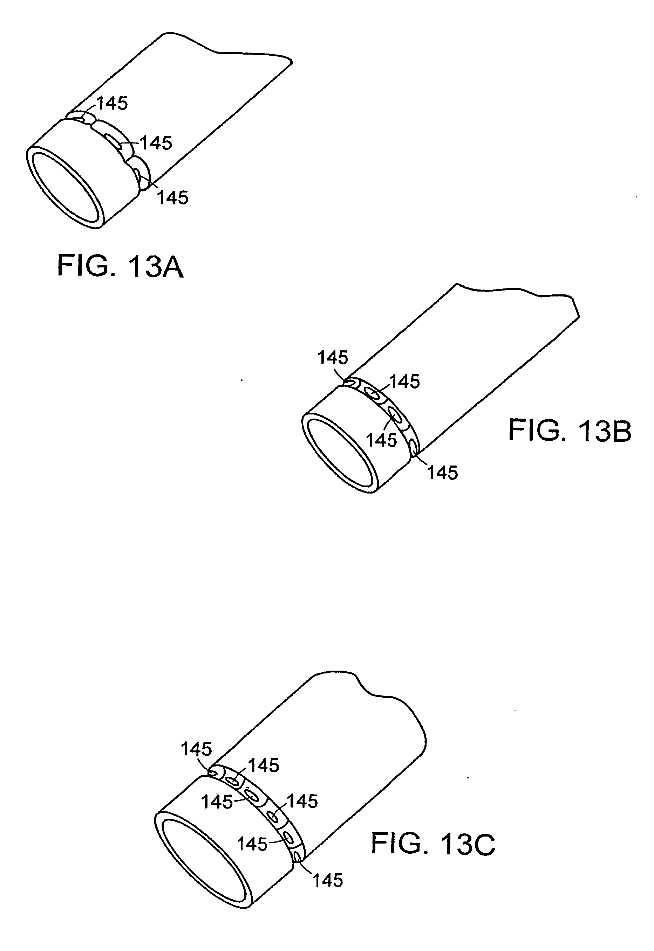

A preferred orientation of the major axis of the ellipse can be obtained by controlling the alignment of the ESD wire and the tube. The process can be repeated around the perimeter of the elongated member 1120 thereby forming an array. A similar method of manufacture can be used to form apertures 145 in a shroud, as previously described. Exemplary nozzles including 6, 8, and 12 aperture arrays are shown in FIGS. 13A-13C.

As shown in FIG. 14, for surface-injection nozzles formed using an elongated member 1120, a plug 1415 can be inserted into the distal end of the elongated member 1120 to prohibit injection of material in a direction perpendicular to the skin surface. The plug 1415 can be contoured to facilitate directing material from the lumen 1110 of the elongated member 1120 toward the apertures 145.

Preferably, any of the embodiments of the surface injection device 100 described herein can be configured in a portable configuration. For example, portable injection device can be configured as a handheld device as shown in FIG. 15. The power source can be provided by a remote power source, such as utility electrical power. Alternatively or in addition the power source can be a self-contained power source, such as a battery.

While this invention has been particularly shown and described with references to preferred embodiments thereof, it will be understood by those skilled in the art that various changes in form and details may be made therein without departing from the scope of the invention encompassed by the appended claims.

## Claims

1. A needle-free transdermal transport device (100) for transferring a substance (135) across a surface of a biological body comprising:
a reservoir (110) for storing the substance (135);
a nozzle (115) having one or more apertures for delivering the substance (135) near the distal end (140) thereof, the nozzle (115) being in fluid communication with the reservoir (110) and adapted to be pressed longitudinally against and depress without piercing, the surface of the biological body; and
an actuator (105) in communication with the reservoir (110) that generates a pressure within the reservoir (110) when activated, **characterized by**:
at least one lateral aperture(145) in the nozzle(115), the aperture being positioned at an angle of at least 45 degrees with respect to a longitudinal axis(210) of the nozzle to allow the substance to expel into the body substantially parallel to the surface of the biological body to produce a shallow injection; and
the actuator injecting the substance laterally through the aperture into the biological body by piercing the surface of the biological body, and producing the shallow injection.

2. The transport device of claim 1, wherein the nozzle (115) comprises a plurality of lateral apertures (145).

3. The transport device of claim 1, wherein the actuator (105) is controllable during an actuation to vary the pressure.

4. The transport device of claim 1, wherein the nozzle (115), is adapted to produce a shallow injection with respect to surface, when the axis of the nozzle (115) is perpendicularly aligned with a surface of the body.

5. The transport device of claim 1, further comprising a retractable shroud (710) in communication with the nozzle (115).

6. The transport device of claim 1, wherein the nozzle (115) comprises a tube with a closed end and having apertures through the side of the tube.

7. The transport device of claim 6, wherein the tube is closed with a plug having an inner surface that directs flow to the apertures (145).

8. A method of manufacturing a nozzle adapted to transfer a substance non- axially to a surface of a body comprising:
providing an elongated member (1120) defining a central lumen (1110);
positioning one end of the elongated member (1120) such that it is in close proximity with an Electro Spark Discharge (ESD) wire (1210); and
energizing the ESD wire (1210) to vaporize the end of the elongated member (1120), thereby forming a non-axial aperture, adapted to transfer the substance to the surface of the biological body.

9. The method of claim 8, wherein energizing further comprises vaporizing using a linear ESD wire (1210), adapted to form an elliptical aperture on the elongated member (1120).

10. The method of claim 9, further comprising controlling the alignment of the ESD wire (1210) and the elongated member (1120) such that a preferred orientation of a major axis of the elliptical aperture can be obtained.

11. The method of claim 8, further comprising repeatedly vaporizing different portions of the end of the elongated member (1120) with an ESD to form an array of apertures between an exterior of the elongated member (1120) and the central lumen (1110).

## Patentansprüche

1. Eine nadelfreie transdermale Transportvorrichtung (100) zur Überführung einer Substanz (135) durch eine Oberfläche eines biologischen Körpers, umfassend:
einen Speicher (110) zum Aufbewahren der Substanz (135);
eine Düse, die eine oder mehrere Öffnungen zur Abgabe der Substanz (135) in der Nähe ihres Distalendes (140) aufweist, wobei die Düse (115) sich im Flüssigkeitsaustausch mit dem Speicher (110) befindet und geeignet ist, in Längsrichtung gegen die Oberfläche des biologischen Körpers gepresst zu werden und diese herabzudrücken ohne sie zu durchlöchern; und
ein mit dem Speicher in Verbindung stehendes Bedienungselement (105), das bei seiner Aktivierung einen Druck im Speicher (110) generiert, **gekennzeichnet durch**:
mindestens eine seitliche Öffnung (145) in der Düse (115), wobei die Öffnung mit einem Winkel von mindestens 45 Grad in Bezug zu einer Längsachse (210) der Düse angeordnet ist, um es der Substanz zu ermöglichen, im Wesentlichen parallel zur Oberfläche des biologischen Körpers in den biologischen Körper einzudringen, um eine flache Injektion zu bewirken; und
die Bedienungseinrichtung, welche die Substanz seitlich **durch** die Öffnung unter Durchdringen der Oberfläche des biologischen Körpers in den biologischen Körper injiziert und eine flache Injektion bewirkt.

2. Transportvorrichtung nach Anspruch 1, wobei die Düse (115) eine Vielzahl von seitlichen Öffnungen (145) umfasst.

3. Transportvorrichtung nach Anspruch 1, wobei die Bedienungseinrichtung (105) während einer Betätigung kontrolliert werden kann, um den Druck zu variieren.

4. Transportvorrichtung nach Anspruch 1, wobei die Düse (115) geeignet ist, eine flache Injektion in Bezug auf die Oberfläche zu bewirken, wenn die Achse der Düse (115) senkrecht zu einer Oberfläche des Körpers angeordnet ist.

5. Transportvorrichtung nach Anspruch 1, wobei die Transportvorrichtung zusätzlich eine in Verbindung mit der Düse (115) stehende einziehbare Abdeckung (710) umfasst.

6. Transportvorrichtung nach Anspruch 1, wobei die Düse (115) ein Rohr mit einem geschlossenen Ende umfasst und Öffnungen durch die Seite des Rohrs aufweist.

7. Transportvorrichtung nach Anspruch 6, wobei das Rohr mit einem Pfropfen verschlossen ist, wobei der Pfropfen eine innere Oberfläche aufweist, die direkt zu den Öffnungen (145) führt.

8. Verfahren zu Herstellung einer Düse, die geeignet ist, eine Substanz nichtaxial zu einer Oberfläche eines Körpers zu überführen, mit den Schritten:
Bereitstellung eines einen zentralen Hohlraum (1110) definierenden länglichen Bauteils (1120);
Positionieren eines Endes des länglichen Bauteils (1120) so, dass es sich in dichter Nachbarschaft zu einem Elektrofunkenentladung(ESD)-Kabel (1210) befindet;
Anlegen einer Spannung an das ESD-Kabels (1210), um das Ende des länglichen Bauteils (1120) zu verdampfen und dabei eine nichtaxiale Öffnung auszubilden, die geeignet
ist, die Substanz zur Oberfläche des biologischen Körpers zu überführen.

9. Verfahren nach Anspruch 8, wobei das Anlegen einer Spannung zusätzlich das Verdampfen unter Einsatz eines linearen ESD-Kabels (1210) umfasst, wobei das Verfahren geeignet ist, eine elliptische Öffnung auf dem länglichen Bauteil (1120) auszubilden.

10. Verfahren nach Anspruch 9, wobei das Verfahren darüber hinaus das Kontrollieren der Anordnung des ESD-Kabels (1210) und des länglichen Bauteils (1120) umfasst, so dass eine bevorzugte Orientierung der Hauptachse der elliptischen Öffnung erreicht werden kann.

11. Verfahren nach Anspruch 8, wobei das Verfahren darüber hinaus ein wiederholtes Verdampfen unterschiedlicher Teile der Endes des länglichen Bauteils (1120) mit einem ESD umfasst, um einen Anordnung von Öffnungen zwischen einer Außenseite des länglichen Bauteils (1120) und dem zentralen Hohlraum (1110) auszubilden.

## Revendications

1. Dispositif de transport transdermique sans aiguille (100) pour transférer une substance (135) à travers une surface d'un corps biologique, comprenant :
un réservoir (110) pour stocker la substance (135) ;
une buse (115) comportant une ou plusieurs ouvertures pour fournir de la substance (135) à proximité de son extrémité distale (140), ladite buse (115) étant en communication de fluide avec le réservoir (110) et apte à être pressée longitudinalement et relâchée, sans perçage, contre la surface du corps biologique; et
un actionneur (105), en communication avec le réservoir (110), qui génère une pression dans le réservoir (110) lorsqu'il est actionné, **caractérisé par** :
au moins une ouverture latérale (145) dans la buse (115), l'ouverture étant positionnée avec un angle d'au moins 45° par rapport à un axe longitudinal (210) de la buse pour permettre que la substance soit expulsée dans le corps sensiblement parallèle à la surface du corps biologique pour produire une injection peu profonde ; et
l'actionneur injectant la substance latéralement, par l'intermédiaire de l'ouverture, dans le corps biologique en perçant la surface du corps biologique et en produisant l'injection peu profonde.

2. Dispositif de transport selon la revendication 1, dans lequel la buse (115) comporte une pluralité d'ouvertures latérales (145).

3. Dispositif de transport selon la revendication 1, dans lequel l'actionneur (105) peut être commandé pendant un actionnement pour faire varier la pression.

4. Dispositif de transport selon la revendication 1, dans lequel la buse (115) est adaptée à produire une injection peu profonde par rapport à la surface, lorsque l'axe de la buse (115) est aligné sur une perpendiculaire à la surface du corps.

5. Dispositif de transport selon la revendication 1, comprenant en outre un flasque (710) rétractable en communication avec la buse (115).

6. Dispositif de transport selon la revendication 1, dans lequel la buse (115) comprend un tube ayant une extrémité fermée et comportant des ouvertures dans le côté du tube.

7. Dispositif de transport selon la revendication 6, dans lequel le tube est fermé par un obturateur ayant une surface intérieure qui dirige le flux vers les ouvertures (145).

8. Procédé de fabrication d'une buse adaptée à transférer non-axialement une substance vers une surface d'un corps, comprenant les étapes suivantes :
prévoir un élément allongé (1120) définissant une lumière centrale (1110) ;
positionner une extrémité de l'élément allongé (1120) de telle sorte qu'elle soit à proximité immédiate d'un fil de décharge d'étincelles électriques (ESD) (1210) ; et
exciter le fil ESD (1210) pour vaporiser l'extrémité de l'élément allongé (1120), formant ainsi une ouverture non axiale, adaptée à transférer la substance vers la surface du corps biologique.

9. Procédé selon la revendication 8, dans lequel l'excitation comprend en outre une vaporisation utilisant un fil ESD linéaire (1210), adaptée à former une ouverture elliptique sur l'élément allongé (1120).

10. Procédé selon la revendication 9, comprenant en outre le contrôle de l'alignement du fil ESD (1210) et de l'élément allongé (1120) de sorte qu'on peut obtenir une orientation préférée de l'axe principal de l'ouverture elliptique.

11. Procédé selon la revendication 8, comprenant en outre une vaporisation répétée de différentes portions de l'extrémité de l'élément allongé (1120) à l'aide d'un ESD pour former un réseau d'ouvertures entre l'extérieur de l'élément allongé (1120) et la lumière centrale (1110).
